# EUROPÄISCHE PATENTANMELDUNG

(11) **EP 1 243 907 A2**
(43) Veröffentlichungstag der Anmeldung: **25.09.2002**
(21) Anmeldenummer: 02005724.6
(22) Anmeldetag: 13.03.2002
(51) Int. Cl.: G01N 1/00, G01N 33/00, G01N 35/08, H01J 49/04

(54) **Verfahren und Vorrichtung zur Probenanalyse**

(30) Priorität: 20.03.2001 DE 10113575
(71) Anmelder: Forschungszentrum Jülich GmbH, 52425 Jülich (DE)
(72) Erfinder: Boner, Markus, 50169 Kerpen (DE)

(57) **Zusammenfassung**

Die Erfindung betrifft ein Verfahren sowie eine Vorrichtung zur Probenanalyse.

Zur Untersuchung von Substanzen werden Proben entnommen und dem jeweils erforderlichen Analyseverfahren zugeführt. Mehrfachmessungen sind für eine hohe Meßgenauigkeit und eine statistisch abgesicherte Auswertung erforderlich.

Die bekannten Meßverfahren weisen folgende Nachteile auf:

Einfachmessungen sind lediglich direkt möglich. Wiederholende Messungen, die zeit und arbeitsaufwendig sind, sind nur durch mehrmalige Injektion nach erneuter Probenahme möglich.

Mit dem erfindungsgemäßen Verfahren und der Vorrichtung ist es nunmehr möglich in kurzer Zeit ohne erneute Probenahme Mehrfachmessungen einer Probe durchzuführen (z.B. 8 Messungen in 2 min). Die Probenmenge kann für den Eintritt in das Analysesystem so reguliert werden, daß die Ergebnisse immer im Meßbereich des Systems liegen.

## Beschreibung

Die Erfindung betrifft ein Verfahren sowie eine Vorrichtung zur Probenanalyse.
Zur Untersuchung von Substanzen werden Proben entnommen und dem jeweils erforderlichen Analyseverfahren zugeführt. Mehrfachmessungen sind für eine hohe Meßgenauigkeit und eine statistisch abgesicherte Auswertung erforderlich.
Analytische Untersuchungen spielen u. a. bei Lebensmitteluntersuchungen eine Rolle. Der Nachweis von unerlaubten Zusätzen ist hier von besonderem Interesse. Hier kann u. a. mit Hilfe der Isotopenzusammensetzung eine Aussage über die Herkunft und das Herstellungsverfahren dieser Lebensmittel getroffen werden.
So kann mit Hilfe der Isotopenanalyse beispielsweise überprüft werden, ob das Kohlendioxid von Schaum- und Perlweinen wie gesetzlich vorgeschrieben ausschließlich aus der Gärung resultiert.
Schaum- und Perlweine sind Veredelungs- oder Bearbeitungsprodukte des Weines mit überhöhtem Kohlendioxidgehalt.
Sie unterscheiden sich im wesentlichen in der Anzahl der Gärungsstufen. Der Kohlendioxidgehalt von Perlwein (Asti, Prosecco frizzante) wird durch eine Gärung gebildet, der des Schaumweins (Sekt) resultiert aus 2 Gärungen. Resultierend aus den Gärungsstufen ist eines der begriffswesentlichen Merkmale von Schaumwein das Vorhandensein von ausschließlich aus der Gärung stammenden Kohlendioxid, das in geschlossenen Behältnissen bei 20°C einen Überdruck von mindestens 3 bar bedingt. Perlweine sind Produkte mit geringerem Überdruck.
Die gesetzliche Auslegung, daß ausschließlich Gärungskohlendioxid enthalten sein darf, kann problematisch für die Herstellung sein, da beim Transvasierverfahren, z.B. der Umfüllung der Tankgärung auf Flaschen, das Vorspanngas (Quell- oder Prozesskohlendioxid) bei unoder gewollter (Druckerhöhung) fehlerhaften Handhabung das Produkt leicht verfälscht.
Solche Produkte, mit teilweisem oder vollständigem Zusatz (letzteres auch als Imprägnierung bezeichnet) von Kohlendioxid, müssen entsprechend gekennzeichnet werden.
Quellkohlendioxid aus Carbonatsedimenten weist ¹³C/¹²C-Isotopenwerte ähnlich der mariner Carbonate auf. In der Zone des Rheinischen Massivs (Eifel, Westerwald, Taunus) einer der produktivsten Quellkohlendioxidzonen Deutschlands, sind ¹³C/¹²C- Isotopenwerte von 0 bis 10‰ zu beobachten. Dagegen ist bei Prozeßkohlendioxid, das überwiegend aus Methanverbrennung dargestellt wird, der ¹³C/¹²C- Isotopenwert bis ca. -50‰ abgereichert.
Zucker von C3-Pflanzen (Weintraube, Zuckerrübe) zeigen Werte von -24 bis -30‰. Das daraus durch Gärung gebildete Kohlendioxid zeigt ähnliche Werte.
Die Messung der ¹³C/¹²C-Isotopenverhältnisse im Kohlendioxid der Schaum- und Perlweine kann die Authentizität des Kohlendioxids nachweisen.

Derzeit werden einfache Gase mit einer modifizierten gasdichten Spritze aus gasdichten Gefäßen entnommen. Das enthaltene Kohlendioxid wird durch eine Kühlfalle, gekühlt mit flüssigem Stickstoff ausgefroren, um andere nicht kondensierbare Gase abzutrennen. Die Falle wird danach durch eine Ethanol-Kühlfalle ersetzt, damit das Kohlendioxid selektiv freigesetzt werden kann. Anschließend kann das Kohlendioxid z.B. über ein Spritzen-Septen-System dem CF-IRMS (Continous Flow Isotope Ratio Mass Spectrometer) zugeführt werden.

Wiederholende Messungen sind bei einem solchen Spritzen-Septen-System nur durch mehrmalige Injektion möglich, die zeit- und arbeitsaufwendig sind. Ist die Zusammensetzung des Meßgases nicht bekannt, so ist eine mehrmalige Injektion mit unterschiedlichen Gasvolumina zwingend, um in den Meßbereich des Massenspektrometers zu gelangen. Eine Volumenentnahme ist mit einer Spritze nur unzureichend kontrollierbar. Im Hinblick auf einen Flaschendruck (z. B. Sekt/Prosecco) von 1 bis 6 bar ist die gute Kontrollierbarkeit und Regulierbarkeit des Entnahmesystems zwingend.
Die Reinigung des Meßgases erfolgt in den derzeitigen Systemen mit arbeits- und zeitaufwendigen Kryoreinigungschritten. Für die massenspektrometrische Analyse, ist eine zeitaufwendige Gaschromatographie des Injektionsgases üblich.

Es ist daher Aufgabe der Erfindung ein Verfahren zu schaffen, mit dem es möglich ist, innerhalb kürzester Zeit eine Probe sowie auch geringe Probenmengen mehrfach mit einer hohen Meßgenauigkeit zu analysieren.

Weiterhin ist es Aufgabe der Erfindung ein Verfahren zu schaffen, welches es erlaubt, spezifische Probenmengen zu entnehmen und während des Meßlaufes Nachregulierungen vornehmen zu können, so daß der Meßbereich des Massenspektrometers immer erreicht wird.

Ausgehend vom Oberbegriff des Anspruchs 1 wird die Aufgabe erfindungsgemäß gelöst mit den im kennzeichnenden Teil des Anspruchs 1 angegebenen Merkmalen. Die Aufgabe wird weiterhin ausgehend vom Oberbegriff des Anspruchs 18 erfindungsgemäß gelöst mit den im kennzeichnenden Teil des Anspruchs 18 angegebenen Merkmalen.
Mit dem erfindungsgemäßen Verfahren und der Vorrichtung ist es nunmehr möglich in kurzer Zeit ohne erneute Probenahme Mehrfachmessungen einer Probe durchzuführen (z.B. 8 Messungen in 2 min). Die Probenmenge kann für den Eintritt in das Analysesystem so reguliert werden, daß die Ergebnisse immer im Meßbereich des Systems liegen.

Vorteilhafte Weiterbildungen sind in den Unteransprüchen angegeben.
Die Zeichnungen zeigen eine beispielhafte Ausführungsform des erfindungsgemäßen Verfahrens und der Vorrichtung.
Es zeigt:
- Fig. 1:: Anordnung Meßsystem
- Fig. 2:: Schaltschema Ventil
- Fig. 3:: schematischer Meßverlauf

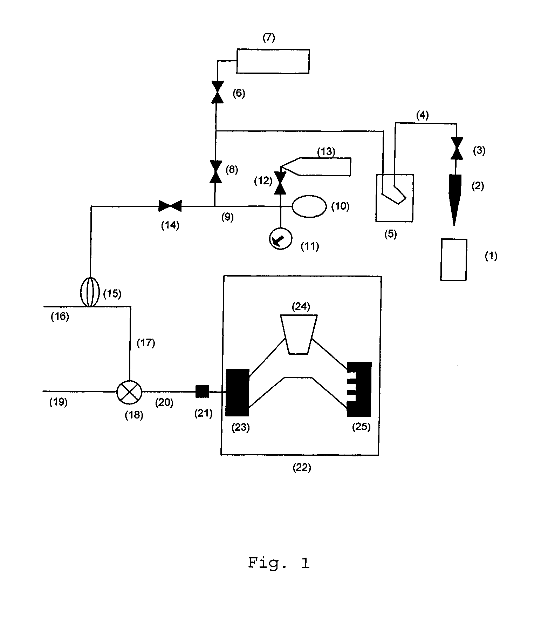

Figur 1 zeigt die schematische Anordnung des Meßsystems für das erfindungsgemäße Verfahren. Aus dem Probengefäß 1 wird mit Hilfe des Entnahmesystems 2 eine Probe entnommen. Das Entnahmesystem 2 mündet in eine mit einem Ventil 3 ausgestattete Leitung 4. Diese Leitung 4 aus dem Entnahmesystem 2 mündet in eine Kryofalle 5. Aus der Kryofalle 5 verläuft die Leitung 4 weiter und gabelt sich t-förmig. Sie ist über ein Ventil 6 sowohl mit einer Vakuumpumpe 7 verbunden als auch über ein weiteres Ventil 8 an eine weitere Leitung 9 angeschlossen. Diese Leitung 9 ist an ein Vorratsgefäß 10 angeschlossen. Weiterhin sind an diese Leitung 9 ein Druckmesser 11 sowie eine über ein Ventil 12 regulierte Gaszufuhr 13 angeschlossen. Die Leitung 9 verläuft über ein Ventil 14 zu einer Drosselkapillare 15. An der Drosselkapillare 15 vereinigen sich die Trägergasleitung 16 mit der Leitung 9, die das Probengas enthält zu einer gemeinsamen Leitung 17, die dann in das 2/4 Wege-Ventil 18 mündet. Am 2/4 Wege-Ventil 18 treffen die Leitung 17 und eine weitere Trägergasleitung 19 zusammen. Aus dem 2/4 Wege-Ventil 18 verläuft eine Leitung 20 über ein Open-Split Interface 21 in das Massenspektrometer 22, welches aus den Komponenten Ionenquelle 23, Elektromagnet 24 sowie Detektoren 25 besteht.

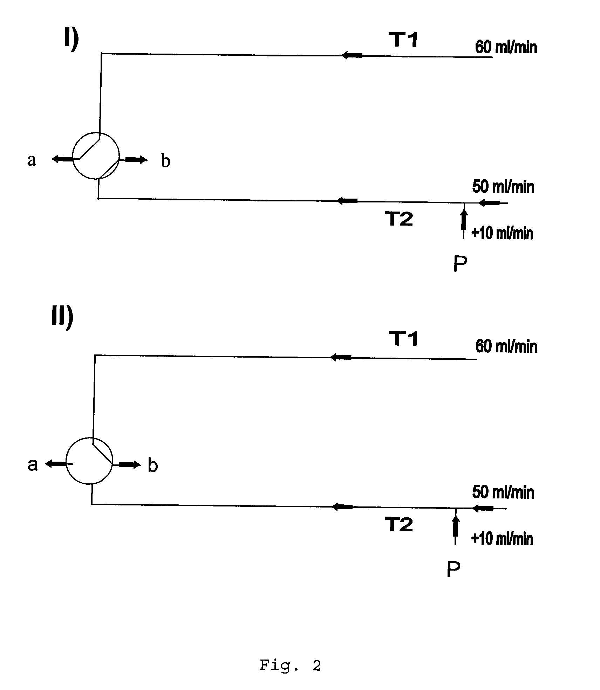

Figur 2 zeigt ein Schaltschema des 2/4 Wege-Ventils 18. Die in Teil I) der Figur dargestellte Position des 2/4 Ventils 18 ermöglicht die Zufuhr von Trägergas T1 zum Analysegerät in Richtung a. Trägergas T2 mit dem enthaltenen Probenstrom P ist hierbei auf Außenatmosphäre in Richtung b geschaltet. Die in Teil II) der Figur dargestellte Position des 2/4 Ventils 18 ermöglicht die Zufuhr der mit dem Trägergas T2 gemischten Probe P zum Analysegerät in Richtung a.

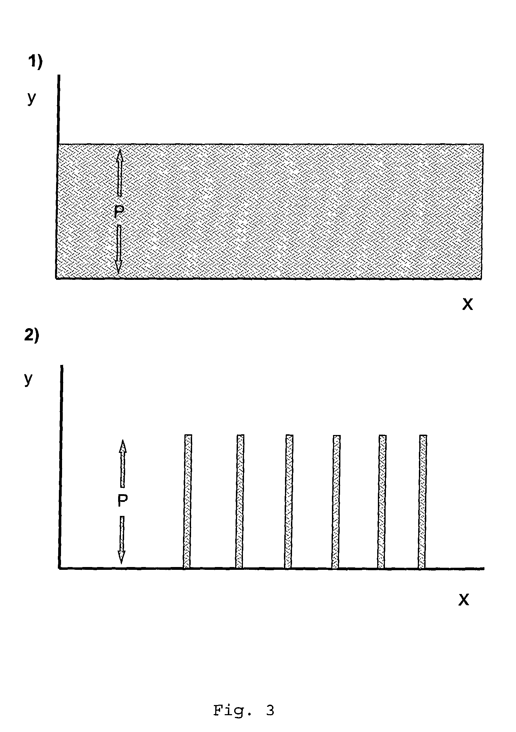

Figur 3 zeigt beispielhaft einen schematischen Meßverlauf. Dabei entspricht die Abszisse X der Zeit in min und die Ordinate Y dem zugegebenen Probenstrom P in ml/min. Bild 1 der Figur 3 zeigt die Messung des kontinuierlichen Probengasstroms ohne Wechselschaltung zwischen Reinträgergasstrom und Probengasstrom. Bild 2 der Figur 3 zeigt die Messung des Probengasstroms mit Wechselschaltung zwischen Reinträgergasstrom und Probengasstrom. Die senkrecht verlaufenden Balken stellen die künstlich erzeugten Meßpeaks dar.

Im folgenden soll die Erfindung beispielhaft beschrieben werden.

Zur Vorbereitung der Messung wird das System mit Hilfe der Vakuumpumpe 7 bis zum Ventil 14 evakuiert. Dabei wird ein Unterdruck im System von beispielsweise 1 mbar erzeugt. Die Evakuierung dient in erster Linie dazu, die störende (Atmosphäre enthält 1% Kohlendioxid) Fremdatmosphäre zu entfernen. Möglich ist jedoch auch das Anlegen eines Unterdrucks von 0,01 bis 100 mbar. Unter dem Begriff "System" soll die gesamte Anordnung aus Analysegeräten, Leitungen, Ventilen, Vorratsbehältern, Reinigungseinheiten, Entnahmevorrichtungen sowie Regulationseinheiten verstanden werden.
Aus dem Probegefäß wird mit Hilfe einer Entnahmevorrichtung 2 eine Probe entnommen. Durch den vorher erzeugten Unterdruck kann die Probe in das System überführt werden. Mit Hilfe des Ventils 3, bevorzugt Feinventil, hinter der Entnahmevorrichtung 2 kann eine kontrollierte Probenzufuhr erfolgen. Dies ist insbesondere dann wichtig, wenn das Probengefäß, wie z. B. eine Sektflasche einen Eigendruck (z. B. 1 bis 6 bar) aufweist. Um die Probe zu reinigen kann sie in eine Kryofalle geleitet werden. Hier werden organische Verunreinigungen sowie Wasser durch Ausfrieren entfernt. Anschließend wird die gereinigte Probe in ein Vorratsgefäß 10 geleitet. Durch die Zufuhr der Probe entsteht eine Druckzunahme. Durch den Druck kann die überführte Probenmenge gesteuert werden. Der Druck kann in einem Bereich von 2 bis 1000 mbar liegen. Das Vorratsgefäß 10 kann ein Volumen von 10 bis 1000 ml aufweisen. Geeignet ist ein Volumen von 250 ml. Mit Hilfe einer Gaszufuhr 13 wird ein Überdruck aufgebaut. Dieser ist über eine Ventil 12 in Verbindung mit einem Druckmesser 11 regulierbar. Der eingestellte Überdruck liegt beispielsweise in einem Bereich von 1,1 bis 2,0 bar. Die Probe wird dadurch mit dem Gas, welches zur Erzeugung des Überdrucks eingesetzt wurde, im Vorratsgefäß 10 vermischt. Dieses Gas sollte mit dem Trägergas welches im Analysegerät eingesetzt wird übereinstimmen, da es mit der Probe zusammen analysiert wird und sonst zur Verfälschung der Meßergebnisse führen würde. Mit Hilfe des so erzeugten künstlichen Vordrucks in Verbindung mit einer Regulationseinheit, z. B. Drosselkapillare ist die Zufuhr der Probe in das Analysegerät gut regulierbar. Dies ist notwendig, wenn der Meßpeak des Probengases unterhalb des Meßbereichs des Analysegeräts liegt. Durch Druckerhöhung kann dann die Zufuhrmenge nachreguliert werden.

Durch diese Meßanordnung ist es möglich eine Probe mehrfach zu analysieren.
Nach Entnahme der Probe und dem Aufbau des Überdrucks werden das Ventil 8 geschlossen und das Ventil 14 geöffnet. Die Probe kann nun zum Analysegerät weitergeleitet werden. Die Zufuhr der Probe aus dem Vorratsgefäß 10 zum Analysegerät wird reguliert z. B. mit Hilfe einer Drosselkapillare 15. Die Drosselkapillare 15 erlaubt einen Volumenstrom der Probe in einem Bereich von 1 ml bis 100 ml pro Minute abhängig vom angelegten Vordruck und verwendeter Drosselkapillare 15. Dieser Volumenstrom wird nun dem Trägergas aus der Trägergasleitung 16 des Continuous Flow Systems zugeführt. Hier wird der Volumenstrom der Probe zu einem kontinuierlichen Volumenstrom aus Trägergas und Probe zusammengeführt. Im folgenden soll unter dem Begriff Strom immer der Volumenstrom der Probe oder des Trägergases verstanden werden.
Bei einer Zuleitung von 10 ml/min Probe zu einem Trägergas mit einem Volumenstrom von 50 ml/min erhält man somit einen Volumenstrom von 60 ml/min Probe-Trägergas. Dieser Proben-Trägergasstrom strömt über ein Open Split Interface in das Analysegerät, z. B. Massenspektrometer und wird dort analysiert. Dabei reguliert ein Wege-Ventil (z. B. 2/4 Wege-Ventil 18) die Zufuhr. Durch Wechselschaltung zwischen Proben-Trägergasstrom und reinem Trägergasstrom (= Reinträgergasstrom) werden im Analysegerät künstliche Peaks erzeugt. Ein Ventil z. B. 2/4 Wege-Ventil 18 steuert dabei die Zufuhr der beiden Ströme zum Analysegerät. Die Meßzeit dieser beiden Volumenströme kann beliebig variiert werden und beträgt beispielsweise 2 Sekunden für den Proben-Trägergasstrom und 10 bis 30 Sekunden für den Reinträgergasstrom. Die vorteilhafte Ausführung des Verfahrens, indem eine Verdünnung des Probenstroms mit Trägergas aus der Trägergasleitung 16 durchgeführt wird, verursacht eine Volumenerhöhung. Die Anzahl der möglichen Mehrfachanalysen wird dadurch zusätzlich erhöht, sowie auch die Möglichkeit während des Meßverlaufs eine Nachregulierung der Probenmenge vorzunehmen.
Weiterhin ist es jedoch auch möglich, die Probe aus dem Vorratsgefäß 10 über die Leitung 9, reguliert durch die Drosselkapillare 15, direkt ohne Verdünnung mit Trägergas aus der Leitung 16, in das Analysegerät zu geben. Dadurch kommt es zu einer direkten Wechselschaltung zwischen Probenstrom und Trägergasstrom. Die Generierung von künstlichen Meßpeaks ist so ebenfalls möglich.

Durch die Wechselschaltung des 2/4 Wege-Ventils 18 wird die Probe nicht durch eine einmalige Analyse untersucht, sondern kann auf kleinere Probenvolumina aufgeteilt werden. Die Probe wird so in Portionen in das Analysegerät gegeben.

Das erfindungsgemäße Verfahren und die Vorrichtung eignen sich beispielsweise auch für die Isotopen-Analyse von Substanzen, die Stickstoff enthalten oder für Untersuchungen von Kohlenmonoxidreingasen.

### Ausführungsbeispiel:

### Messung des ¹²C/¹³C-Isotopenverhältnisses im Kohlendioxid von Schaumwein (Sekt)

Aus dem Probengefäß 1, z. B. einer Sektflasche, wird mit Hilfe eines Entnahmesystems 2, z. B. Sektprüfer, aus dem Gasraum eine Probe entnommen. Zuvor wurde das gesamte System der Meßanordnung bis zum Ventil 14, welches während der Evakuierung geschlossen ist, mit Hilfe der Vakuumpumpe 7 auf einen Unterdruck von beispielsweise 1 mbar evakuiert. Mit Hilfe des erzeugten Unterdrucks wird die Gasphase des Probengefäßes in das Vorratsgefäß 10 überführt. Dieses kann ein Volumen von 10 bis 1000 ml haben. Besonders geeignet ist ein Volumen von 250 ml. Das Ventil 3, welches bevorzugt ein Feinventil ist, ermöglicht die kontrollierte und langsame Überführung der Gasprobe aus dem Probengefäß 1 in das System. Durch die Zufuhr des Gasprobe kommt es zu einem leichten Druckanstieg in dem System (ca. 50 mbar). Die Gasprobe wird zur Reinigung, insbesondere zum Ausfrieren von Wasser und organischen Verbindungen, durch die Kryofalle 5 geleitet. Nach der Probenahme werden die Ventile 3, 6, 8 geschlossen und mit Hilfe der Zufuhr von Trägergas z. B. Helium ein Überdruck von 1,5 bar aufgebaut. Mit Hilfe des so erzeugten Überdrucks im System ist es möglich, die Übertrittsmenge der Probe in das Massenspektrometer-System zu regulieren, d.h. sind die Meßpeaks unterhalb des Meßbereiches des Massenspektrometers, so kann dies mit einfacher Druckerhöhung nachreguliert werden. Darüber hinaus wird die Gasprobe mit dem Trägergas z. B. Helium vermischt. Als Trägergas eigenen sich insbesondere Edelgase, wie z. B. He und Ar aber auch Wasserstoff. Durch Öffnen von Ventil 14 wird die Gasprobe aus der Leitung 9 dem Trägergas aus der Trägergasleitung 16 des Continuous Flow System zugeführt. Die Drosselkapillare 15 ermöglicht die kontrollierte Zufuhr einer geringen Menge an Gasprobe in einem Bereich von 1 bis 100 ml in dem Trägergas zum Analysegerät. Besonders geeignet ist eine Zufuhr von 10 ml/min. Bei einer Zufuhr von 10 ml/min Gasprobe ist eine Zufuhr von Trägergas 16 von 50 ml/min besonders geeignet. Es liegt nun ein kontinuierlicher Proben-Trägergasstrom mit einem Volumenstrom von 60 ml/min vor. Der Reinträgergasstrom sollte einen entsprechenden Volumenstrom aufweisen. Dieser stellt das Reingas/Nullgas dar, das dem Massenspektrometer 22 im entsprechender Ventilschaltung des 2/4 Wege-Ventils 18 zugeführt wird.

Durch die Wechselschaltung zwischen Proben-Trägergasstrom und Reinträgergasstrom wird die Generierung von künstlichen Schaltpeaks ermöglicht (s. Fig. 3). Die Meßzeit des Proben-Trägergasstrom und des Reinträgergasstroms kann beliebig variiert werden, indem je nach Bedarf das 2/4 Wege-Ventil 18 umgeschaltet wird (s. Fig. 2). Die Meßzeit des Proben-Trägergasstroms kann beispielsweise 2 Sekunden betragen, während sie für den Reinträgergasstrom 20 Sekunden betragen kann. Entsprechend bleibt das 2/4 Wege-Ventil 18 für 2 Sekunden in der Position, in der der kontinuierliche Proben-Trägergasstrom aus Leitung 17 durch Leitung 20 in das Massenspektrometer 22 gelangt und für 20 Sekunden in der Position in der der Reinträgergasstrom aus Leitung 19 durch die Leitung 20 in das Massenspektrometer 22 gelangt. Es ist möglich die Schaltzeit für den Reinträgergasstrom in einem Bereich von 2 bis 1000 Sekunden zu variieren.

## Patentansprüche

1. Verfahren zur Probenanalyse,
**dadurch gekennzeichnet,**
**daß** Mittel eingesetzt werden, welche die Zufuhr des Probenstroms zum Analysegerät portionieren.

2. Verfahren nach Anspruch 1,
**dadurch gekennzeichnet,**
**daß** eine Wechselschaltung zwischen Probenstrom und Reinträgergasstrom durchgeführt wird.

3. Verfahren nach einem der Ansprüche 1 bis 2,
**dadurch gekennzeichnet,**
**daß** ein Wegeventil eingesetzt wird.

4. Verfahren nach einem der Ansprüche 1 bis 3,
**dadurch gekennzeichnet,**
**daß** ein 2/4-Wege-Ventil (18) eingesetzt wird.

5. Verfahren nach einem der Ansprüche 1 bis 4,
**dadurch gekennzeichnet,**
**daß** vor Probenahme an das System ein Unterdruck angelegt wird.

6. Verfahren nach einem der Ansprüche 1 bis 5,
**dadurch gekennzeichnet,**
**daß** eine Vakuumpumpe (7) eingesetzt wird.

7. Verfahren nach einem der Ansprüche 1 bis 6,
**dadurch gekennzeichnet,**
**daß** die Probe gereinigt wird.

8. Verfahren nach einem der Ansprüche 1 bis 7,
**dadurch gekennzeichnet,**
**daß** eine Kryofalle (5) eingesetzt wird.

9. Verfahren nach einem der Ansprüche 1 bis 8,
**dadurch gekennzeichnet,**
**daß** die Probe in ein Vorratsgefäß (10) geleitet wird.

10. Verfahren nach einem der Ansprüche 1 bis 9,
**dadurch gekennzeichnet,**
**daß** nach Probenahme ein Überdruck an das System angelegt wird.

11. Verfahren nach einem der Ansprüche 1 bis 10,
**dadurch gekennzeichnet,**
**daß** die Probe mit einer Trägergas versetzt wird.

12. Verfahren nach einem der Ansprüche 1 bis 11,
**dadurch gekennzeichnet,**
**daß** Helium eingesetzt wird.

13. Verfahren nach einem der Ansprüche 1 bis 12,
**dadurch gekennzeichnet,**
**daß** der Volumenstrom der Probe reguliert wird.

14. Verfahren nach einem der Ansprüche 1 bis 13,
**dadurch gekennzeichnet,**
**daß** eine Drosselkapillare 15 eingesetzt wird.

15. Verfahren nach einem der Ansprüche 13 bis 14,
**dadurch gekennzeichnet,**
**daß** ein Volumenstrom von 2 bis 100 ml eingestellt wird.

16. Verfahren nach einem der Ansprüche 1 bis 15,
**dadurch gekennzeichnet,**
**daß** ein Continous Flow System eingesetzt wird.

17. Verfahren nach einem der Ansprüche 1 bis 16,
**dadurch gekennzeichnet,**
**daß** als Analysegerät ein Massenspektrometer (22) eingesetzt wird.

18. Vorrichtung zur Probenanalyse,
**gekennzeichnet durch**
Mittel, welche die Zufuhr des Probenstroms zum Analysegerät portionieren.
